(19) Europäisches Patentamt
European Patent Office
Office européen des brevets

(11) EP 1 551 903 B1

(12) EUROPEAN PATENT SPECIFICATION

(45) Date of publication and mention of the grant of the patent:
**21.03.2007 Bulletin 2007/12**

(21) Application number: **03772676.7**

(22) Date of filing: **17.10.2003**

(51) Int Cl.:
***C08G 73/00*** *(2006.01)* ***C02F 1/50*** *(2006.01)*
***C02F 1/56*** *(2006.01)* ***C08G 73/02*** *(2006.01)*

(86) International application number:
**PCT/IT2003/000643**

(87) International publication number:
**WO 2004/052961 (24.06.2004 Gazette 2004/26)**

# (54) STERILIZING POLYMERS AND PREPARATION AND USE THEREOF

STERILISIERENDE POLYMERE UND DEREN HERSTELLUNG UND VERWENDUNG

POLYMERES DE STERILISATION ET PROCEDE DE FABRICATION ET D'UTILISATION CORRESPONDANT

(84) Designated Contracting States:
**AT BE BG CH CY CZ DE DK EE ES FI FR GB GR HU IE IT LI LU MC NL PT RO SE SI SK TR**

(30) Priority: **18.10.2002 IT RM20020529**

(43) Date of publication of application:
**13.07.2005 Bulletin 2005/28**

(73) Proprietor: **Progetto Idea S.P.A.**
**00186 Roma (IT)**

(72) Inventors:
- **STAINO, Franco**
  **00186 Roma (IT)**
- **KUZNETSOV, Oleg**
  **103055 Mosca (RU)**

(74) Representative: **Germinario, Claudio et al**
**Società Italiana Brevetti S.p.A.**
**Piazza di Pietra 39**
**00186 Roma (IT)**

(56) References cited:
**WO-A-02/083572**
**RU-C- 2 142 451**
**SU-A- 1 616 898**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Field of the Invention

The invention relates to a novel method of polymerizing alkylenediamine- and guanidine-based branched and/or crosslinked copolymers, the copolymers thus obtained, the compositions containing them as well as the use thereof mainly as flocculating, sterilizing and absorbing agents in the treatment of waters, gases or solid materials.

Prior Art

Hexamethylenediamine- and guanidine-based copolymers, like polyhexamethyleneguanidine chloride or sulfate are described in the state of the art as antiseptic agents suitable for water treatment.

The maximum concentration of polyhexamethyleneguanidine phosphate and its homologues in drinking water has been defined on the basis of the studies carried out at the Institute of Human Ecology and Environmental Hygiene of the Sechenov Moscow Medical Academy. In view of its hygienic-toxicological characteristics, the substance is non-toxic up to the maximum concentration of 8.0 mg/l, whereas its residual concentration cannot exceed 1.5 mg/l, without varying the organoleptic characteristics of the water with foaming. For this reason, the optimal concentration of the polyhexamethyleneguanidine phosphate homologues, used for water sterilization and synthesized by any method, cannot exceed the 1.5 mg/l.

A particularly interesting salt for purifying and sterilizing water is the polyhexamethyleneguanidine chloride.

A synthesis method of polyhexamethyleneguanidine chloride is described in Pat. SU 1616898 BI N48, 1990. The method provides the polycondensation of guanidine hydrochloride and hexamethylenediamine. Guanidine hydrochloride synthesis takes place firstly through the melting of dicyanodiamide with ammonia chloride at a 180°C temperature and subsequently through the introduction of hexamethylenediamine in molten state hydroguanidine chloride, all in a single step. The structural formula of guanidine chloride, synthesized with this method, is as follows:

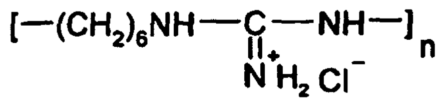

The drawback of the method lies in the heavy losses of hexamethylenediamine during the polycondensation, since hexamethylenediamine in the vapor state is lost along with the produced ammonia. The losses need compensating by increasing the amount of starting substances and, in particular, by using hexamethylenediamine in excess with respect to guanidine chloride. The other drawback of the method lies in the formation of an intermediate product containing cyanuric acid derivatives - ammelides and ammeline. The derivatives do not transform during the reaction and are found in the final product. Hence, the main drawback is that polyhexamethyleneguanidine chloride exhibits worse hygienic qualities with respect to analogous substances used to sterilize water. These drawbacks add up to the intrinsic properties of the substance itself, i.e. toxicity and capability of altering the organoleptic characteristics of water, generally less satisfactory then the characteristics of the phosphate homologue. In fact, the polyhexamethyleneguanidine chloride synthesized in accordance with the method described in Pat.SU1616898 has a maximum non-toxic concentration of 2.0 mg/l, whereas its residual concentration cannot be greater than 1.0 mg/l without altering the organoleptic characteristics of the water, with foaming. Therefore, the optimum concentration value of this polyhexamethyleneguanidine salt employed for sterilizing water cannot exceed the 1.0 mg/l. On the other hand, the effective dosage of the substance in water sterilization and purification is of 1.0 mg/l during 1 hour. This means that the sterilizing concentration is practically equal to the maximum non-toxic concentration. This fact makes it impossible to use the polyhexamethyleneguanidine chloride, synthesized according to the method reported in Pat.SU-1616898, in industrial-scale water potabilisation and restricts it to the preparation of water for technical requirements.

Pat. RU 2144024, published in 2000, describes a synthesis method of a polyhexamethyleneguanidine phosphate exhibiting bactericidal properties, through polycondensation of hexamethylenediamine and of guanidine carbonate at a 110-165°C temperature, and subsequent treatment with orthophosphoric acid. Two are the drawbacks of this method: the low effectiveness of the polymer obtained in water sterilization and the presence of a fraction toxic towards human organism. The first drawback is due to the high content of insoluble substances, as side products of the polycondensation. The high content of these substances is due to the fact that the molar ratio between the hexamethylenediamine and the guanidine carbonate, joined solely in a linear chain, is of2 (1:0.5) during the polycondensation. The synthesis occurs in the presence, as diluent, of toluene that has limited solubility and, upon separation from the final product, leaves an insoluble fraction. This negatively influences water sterilization. In order to be able to potabilise water the effective dosage

of the copolymer synthesized according to Pat.RU 2144024 is of 2-5 mg/l, which significantly exceeds the maximum non-toxic concentration allowed for the substances of this class in drinking water. The other drawback of the method lies in the residual presence of 1% hexamethylenediamine (starting substance not participating to the synthesis). Hexamethylenediamine is a high-toxicity substance, belonging to the second class of toxic substances, and the limit of its tolerable concentration in water is of 0.01 mg/l. With an effective dosage of polyhexamethyleneguanidine phosphate of 1.5 mg/l required to condition water and with the concentration of 1% hexamethylenediamine in the copolymer, a concentration of the latter of 0.015 mg/l is obtained, which is 1.5 times higher than the limit value tolerable in water. Therefore, the hexamethylene guanidine phosphate synthesized according to the method of Pat.RU 2144024 cannot be used to potabilise water.

The known method providing the results closest to those researched and obtained by the present inventors is described in the Russian Pat. RU-2142451 published on 12.10.1999. The method provides the synthesis of a polyhexamethyleneguanidine phosphate, having the antiseptic properties, through the reaction between the polyhexamethyleneguanidine salt and a phosphate-containing substance, under controlled temperature. The final product of the synthesis is separated from the secondary product, washed and dried. The structural formula of guanidine phosphate, synthesized by this method, is as follows:

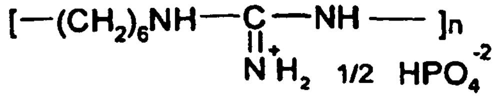

With this method a polyhexamethyleneguanidine phosphate is obtained, having low molecular weight, whose antimicrobial properties and flocculating power are unsatisfactory. The minimum effective antibacterial concentration of the polymer synthesized according to this method is of10-15 mg/l. This remarkably exceeds the maximum concentration tolerable in drinking water. Moreover, concerning the water potabilisation process, the polymer synthesized according to Pat.RU 2142451 exhibited no flocculating power in river water clarification tests.

Lastly, in literature there are no examples of use of polyhexamethyleneguanidine salts for the purification and sterilization of air in living and industrial environments or the sterilization of solid materials and surfaces thereof.

Scope of the present invention is to produce substances more effective than the known ones in the prevention and in the treatment of biological contamination.

In particular, scope of the invention is to produce, through an appropriate synthesis method, polymer substances having, with respect to the prior art polymers, an improved applicability in processes of water sterilisation and potabilisation, of waste water purification, of air in living, industrial or hospital environments purification and sterilisation and in the sterilisation and antifouling treatment of solid materials and surfaces thereof.

Summary of the invention

The present invention is based on a novel synthesis method for producing novel alkylenediamine, such as hexamethylenediamine, and guanidine based copolymers. This method allows the production of branched and/or crosslinked copolymers in form of salt or of free base. The copolymers at issue, when in form of phosphate, are generically referred to as 'Flogucide'.

In the preferred embodiment, the preparation process provides the reaction between guanidine chloride and hexamethylenediamine and it is conducted in various subsequent stages ensuring a more effective use of the various reagents, in particular of hexamethylenediamine, and the attainment of soluble or insoluble copolymers comprising linear chains and branched and/or crosslinked chains.

Such copolymers exhibit flocculating, sterilizing in a broad sense and metal ion-binding properties. These characteristics, which are absent or more limited in prior art products, make the copolymers of the invention particularly effective in the sterilisation and potabilisation of water, the purification of waste water, the purification and sterilisation of gases and specifically of air in living and industrial environments and in health and hospital structures, and in the sterilisation of solid materials and surfaces thereof, as well as in the prevention of biological contamination and of corrosion due thereto.

In accordance with the above, the main object of the invention is a preparation process of branched and/or crosslinked copolymers in which molten state hexamethylenediamine is uniformly added over a period of time of 1.5 to 3 hours to equimolecular, stoichiometric amounts of molten state guanidine chloride and the mixture kept at the melting temperature of the mass under stirring from 3 to 5 hours;

then, additional amounts of molten hexamethylenediamine resulting in a molar excess of hexamethylenediamine with respect to the guanidine chloride of 0.05-0.50 (5%-50%) are added to the molten mass holding the same temperature during a maximum period of time of 60 min;
lastly, the temperature is increased to about 250°C and held thereat until free ammonia production is over, and wherein all the steps provide the condensation and the reintroduction of the hexamethylenediamine vapors in the reaction mixture.

Optionally, the process provides additional steps in which

d) the chloride of the copolymer thus obtained is cooled and granulated and/or
e) converted into another salt or into the corresponding basic form.

The guanidine chloride used in the method of the invention is produced by mixing guanidine or guanidine carbonate or cyanoguanidine or mixtures thereof to equimolecular amounts of ammonium chloride and heating the mixture thus obtained to a temperature of about 200°C, until formation of molten state guanidine chloride.

Preferably, the process of the invention provides the conversion of the chloride of the copolymer into another salt, such as phosphate, sorbate, stearate, dehydroacetate.

Object of the invention is also a process in which the chloride is converted into phosphate through reaction with an ammonium hydro-orthophosphate solution produced by absorption of the ammonia released during the polymerization process in an orthophosphoric acid solution until neutralization.

In the process according to the invention a soluble cationic copolymer is produced using a molar excess of hexamethylenediamine with respect to guanidine chloride of 0.05-0.15, whereas an insoluble cationic polymer having characteristics of a gel swelling in water is obtained using a molar excess of hexamethylenediamine with respect to the guanidine chloride of 0.3-0.50.

Advantageously, the polymerization process comprising a further step of crosslinking carried out with epichlorohydrin or equivalent crosslinking agent causing an increase of the viscosity and/or of the molecular weight.

Further objects of the invention are the branched and/or crosslinked cationic copolymers obtainable through the abovedescribed process. Such copolymers are in form of salts like chloride, phosphate, sorbate, stearate, or dehydroacetate, are soluble in an aqueous medium and exhibit a maximum viscosity of 0.21 dl/g, or are in form of gel insoluble and swelling in an aqueous medium, or in form of free base.

Other objects of the invention are flocculating and/or sterilizing and/or heavy metal ion-binding compositions for water purification and water potabilisation. These compositions contain the cationic copolymer and optionally a suitable excipient comprising one or more diluents, adjuvants or supports and in particular compositions for water potabilisation in which the copolymer is in form of phosphate salt.

Another object of the invention is represented by sterilizing, antifouling and anticorrosion compositions for the treatment of materials or solid surfaces, comprising the copolymer in cationic form or in form of a corresponding base and a suitable excipient.

These compositions are in form of solution, emulsion, suspension, gel, paste or other formulation capable of impregnating said materials or of forming decorative or protective coatings on said surfaces and can be marketed as paints, enamels, latexes or resins.

Said compositions exhibit bactericidal, antiviral, antimycotic or antifouling and anticorrosion activities.

Other objects of the invention are solid materials treated with one of the above compositions. The material may be glass, cellulose, paper, fabric, coal, plastic or other solid polymer, metal, wood, hides or building material. Preferably, the material will be a filtering material suitable for the sterilizing filtration of liquids or gases, or a cationic resin consisting of the insoluble copolymer in form of gel.

Other objects of the invention are articles made with or comprising these materials, e.g. filtering elements suitable for the treatment of gases or liquids, containers or piping for the transport of waters or of air, hides, metallic items or building elements.

Methods of purifying, sterilizing and potabilising waters, methods of sterilizing materials or solid surfaces, antifouling methods for the prevention and the treatment of biological contamination.

## Detailed description of the invention

The substances according to the invention are novel synthetic copolymers, obtained through a novel method of polymerisation carried out using particular reaction conditions.

The copolymer molecule is produced by polymerization of two comonomers: the guanidine salt and a C2-C10 alkylenediamine. The guanidine group confers to the copolymer the characteristics of a cationic flocculating agent. On the other hand, the alkylenediamine group, when comprising from 5 to 8 methylene ($-CH_2-$) groups, exhibits bactericidal, antiviral, antimycotic and antifouling activity. Hexamethylenediamine is the preferred alkylenediamine, it being provided with the most effective activity generically defined as sterilizing and antifouling.

The guanidine phosphate group, present in the preferred form of the copolymer of the invention, is a natural, endogenous and highly energetic substance.

The hexamethylene group is the hexamethylenetetramine - urotropine base, a widely known antiseptic substance.

Hence, the copolymers according to the invention are made of structure elements physiological to the human body and therefore non-toxic.

In fact, it has been observed that the phosphate of the copolymers of the invention (Flogucide) is largely compatible with human and animal organism, and that its therapeutic index reflects a toxicity towards microorganisms 3000 times higher with respect to the toxicity towards human or animal beings.

The copolymers at issue are obtained by reaction between the guanidine chloride and the hexamethylenediamine through a multistep process.

For the preparation of guanidine chloride, equimolar amounts of guanidine $[(H_2N)_2C{=}NH]$ and ammonium chloride $[NH_4Cl]$, are mixed, and the mixture heated to a melting temperature thereof, i.e. of from 180°C to 220°C, preferably up to 200°C. Then, the reaction mixture is kept at the same temperature to evaporation of the ammonia and formation of molten state guanidine chloride $[(H_2N)_2C{=}N^+H_2Cl^-]$ . The reaction is illustrated hereinafter:

$$(H_2N)_2C = NH + NH_3HCl \rightarrow (H_2N)_2C = N^+H_2Cl^- + NH_3$$

In this first step of the polycondensation process, the guanidine may partly or totally be replaced by equivalent substances like guanidine carbonate $[(H_2N)_2C{=}NH \times {}^1/_2H_2CO_3]$ and dicyanodiamide $[(H_2N)_2C{=}N{-}C{=}N]$ used alone or in a mixture at different ratios. These substances are admixed with or without guanidine at any ratio therebetween and used, in the first step of the polycondensation, always keeping unvaried the molar ratio between the mixture of these guanidine derivatives and the ammonium chloride.

The reaction between guanidine and ammonium chloride produces free ammonia, whereas the reaction between guanidine carbonate and ammonium chloride produces carbon dioxide. On the contrary, the reaction between dicyanodiamide usually ends without production of gaseous side products.

The second step of the process consists in the reaction between molten state alkylenediamine, preferably hexamethylenediamine $[H_2N(CH_2)_6NH_2]$, and molten state guanidine chloride, as obtained in the preceding step and holding the same temperature thereof.

The molten hexamethylenediamine, obtained by preheating at a 60°-80°C temperature, is added to the molten guanidine chloride in two subsequent stages. In the first stage, there is introduced, during a period of time of 1.5 to 3 hours, an amount equimolar with respect to the amount of guanidine used; The reaction mixture thus obtained is kept under stirring from 3 to 5 hours.

In the second stage, additional amounts of molten hexamethylenediamine, resulting in a molar excess with respect to the guanidine chloride of0.05-0.50/1, i.e. a percent excess of from 5% to 50%, are added to the molten mass holding the same temperature during a maximum period of time of 60 min. This addition results in a total molar ratio between hexamethylenediamine and guanidine chloride of1.05-1.5:1.

The two reaction steps are carried out in a reactor provided with means for recovering the hexamethylenediamine vapors produced and released during heating. Such means consist in means of condensation and reintroduction of the condensed substance into the reaction mixture.

In the final step of the process, upon introduction of the entire amount of hexamethylenediamine, the temperature is increased to 230-270°C, preferably up to 250°C, and held thereat for from 7 to 15 hours, preferably for 10 hours, and anyhow until gaseous-state ammonia production and release are over.

The interaction of guanidine with ammonium chloride at a 200°C temperature, the introduction of hexamethylenediamine in two subsequent steps with vapor condensing and recycling and the increasing of the temperature up to 250° C during the second step of the synthesis, ensure a maximum yield of use of hexamethylenediamine in the polycondensation, as well as the purity of the synthesized product and the molecular weight thereof.

The increase of the excess of hexamethylenediamine on the equimolecular amount, in the range of from 0.05 to 0.15: 1, leads to the increase of the branching and interchain crosslinking of the linear chains and respectively to the increase of the intrinsic viscosity of the product obtained, without however altering the solubility thereof.

The intrinsic viscosity [η] is an empirical parameter that is measured in dl/g. Said parameter may be calculated according to the following equation:

$$[\eta] = \lim_{C \to 0} [\eta - \eta_s / \eta_s C]$$

where [η] is the intrinsic viscosity, η the solution viscosity, $\eta_s$ the solvent viscosity and C the solution concentration

expressed in g/dl.

The maximum value of the abovementioned intrinsic viscosity is of 0.21 dl/g. This corresponds to the product obtained operating with a ratio of hexamethylenediamine with respect to the guanidine of1.15:1 (15% excess). When the excess is greater and the intrinsic viscosity exceeds the 0.21 dl/g, in the mixture of the products obtained there will appear an increasing fraction of water-insoluble gel and the decrease of the final product volume, respectively. The further increase of the molar ratio between hexamethylenediamine and guanidine up to 1.25:1 (25% excess) results in a soluble final product with the intrinsic viscosity of 0.36 dl/g but containing a large fraction of insoluble gel. Lastly, when the molar excess of hexamethylenediamine is of from 30% to 50% with respect to the guanidine, the copolymer obtained will appear in form of insoluble gel at different levels of in-water swelling, and with the characteristics of a ion-exchange resin added to the bactericidal ones.

Since the formation of gel during polycondensation leads to the clogging of the apparatuses, the use of molar excesses of hexamethylenediamine with respect to the guanidine of from 0.15:1 to 0.5:1 (15% to 50%) will preferably require the presence of a diluent like, e.g., polyethylene glycol. It has to be pointed out that the polycondensation product loses its solubility also for molar excesses of hexamethylenediamine (e.g., 0.15-0.17:1) if the introduction of molten state hexamethylenediamine is carried out in a single stage, as described in SU-1616898.

The intrinsic viscosity and the molecular mass of the soluble copolymer produced by the polycondensation can be further increased, without altering the solubility thereof, through crosslinking reaction with a suitable crosslinking agent. E.g., the polycondensation product could be treated with an equimolar amount of epichlorohydrin operating in an aqueous solution in the presence of a basic substance. The molecular mass of the soluble copolymer thus obtained depends on the molecular mass of the polycondensation product and on the ratio with the crosslinking substance. In the course of the testing there have been identified the conditions for obtaining an in-water soluble copolymer having a high molecular weight and with an intrinsic viscosity of1.0-1.3 dl/g as described in the examples.

While not wishing to bound the invention to specific theories, the present inventors succeeded at defining that the novel characteristics of the copolymers obtained in accordance with the invention stem from the involvement in the polycondensation reaction of the third amine group of the guanidine chloride. Such an involvement, which is due to the use of an excess of hexamethylenediamine and to the specific operating conditions, causes the increase of the branching and/or of the crosslinking of the linear chains with the entailed enhancement of the sterilizing and flocculating properties.

Hence, it is considered that the different reaction stages observed above elapse according to the reaction schemes illustrated below.

In the first step, equimolecular amounts of guanidine chloride and Hexamethylenediamine react according to the following scheme: $n\ (H_2N)_2C{=}N^+H_2Cl^- + nH_2N(CH_2)_6NH_2 \rightarrow [-(CH_2)_6NH\text{-}C(=N^+H_2Cl^-)\text{-}NH]_n + 2nNH_3\uparrow$

The introduction in the reaction mixture of an excess of hexamethylenediamine, under conditions of recovery of the vapors thereof, and the increase of the temperature up to 250°C cause the involvement of the third amine group of the guanidine according to the following scheme:

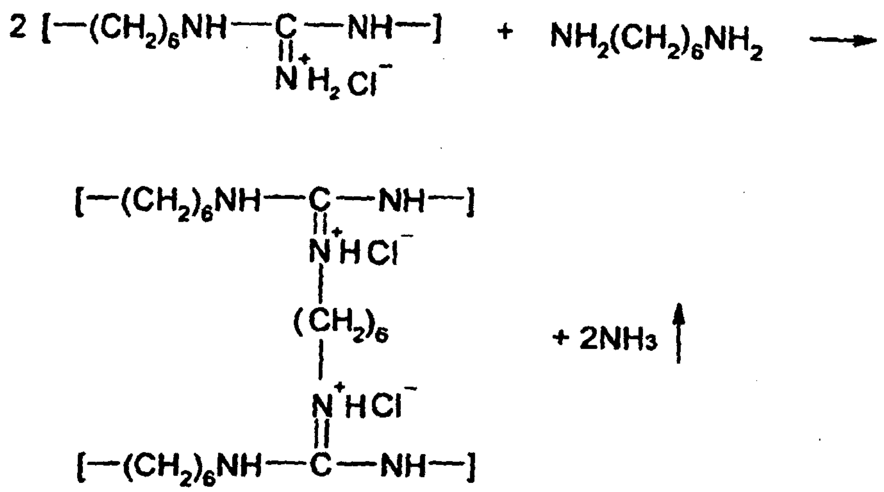

The insertion of the third amine group of guanidine is confirmed by the studies of the distribution of the molecular weight of the copolymer, by the low content of the free hexamethylenediamine (even though present quantitatively in excess with respect to the guanidine), as well as to the amount of ammonia actually released, corresponding to the theoretical amount computed on the basis of the aboveillustrated reaction scheme.

The product obtained through the above polycondensation process, called Polybiocide, is in form of chloride

and can already be used as such in accordance with the present invention. For this purpose, the copolymer is cooled to room temperature until solidification thereof and mechanically granulated.

However, in a preferred embodiment, the chloride salt is replaced by other salts more suitable for the different applications, such as phosphate, sorbate, stearate or dehydroacetate, or it is transformed into the corresponding base through treatment with strongly basic solutions, like alkaline hydroxide solutions. Phosphate is the salt having the widest applicability, and therefore it is preferred. For the preparation of the phosphate, the chloride of the copolymer is converted into the corresponding base, which is subsequently treated with concentrated orthophosphoric acid. In an alternative method, the chloride of the copolymer in granulated form is diluted in water until reaching a 50% solution, then reacted with a dibasic ammonium phosphate - $(NH_4)_2HPO_4$ - solution for about 1 hour at a 40-60°C temperature.

Advantageously, the ammonium sulfate solution is obtained through recovery of the ammonia released during the polycondensation process and conveyed into an aqueous solution of 33-35% orthophosphoric acid using a 2:1 ratio and operating at a 60-80°C temperature, to complete neutralization of the acid and formation of ammonium hydro-ortophosphate - $(NH_4)_2HPO_4$ - according to the reaction:

$$H_3PO_4 + 2NH_3 \rightarrow (NH_4)_2HPO_4$$

Under these conditions there occurs the substitution reaction of the $Cl^-$ ions with the $HPO_4^{2-}$ ones in the polycondensation product.

Considering the length of the bonds formed, the substitution reaction of the chloride ions with phosphate ions occurs between two contiguous groups belonging both to the linear chains and to the cross chains, as illustrated hereinafter:

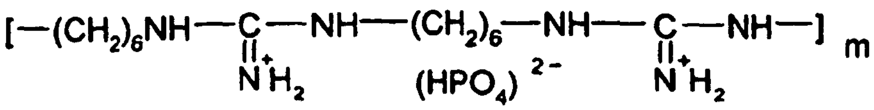

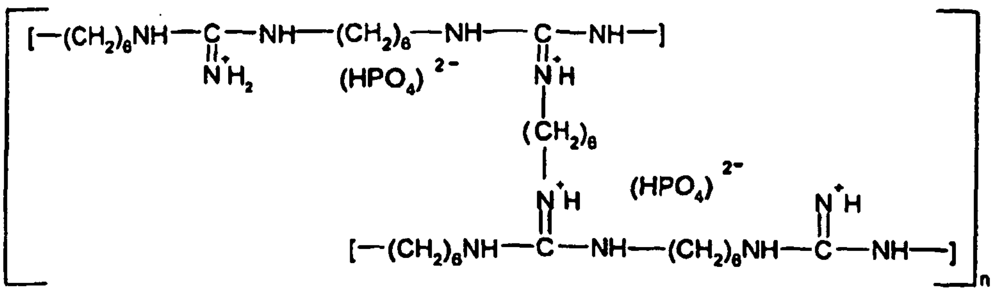

At the end of the substitution reaction conducted at 40°-60°C, the reaction mixture is left to cool to room temperature until separation of two phases: the top one containing the aqueous solution of $NH_4Cl$ and the bottom one containing the aqueous solution of copolymer, which therefore is isolated.

Preferably, the exchange reaction of the chloride ion with the phosphate ion is repeated one or more times. For this purpose, the copolymer solution obtained in the preceding step is diluted with water to the starting volume, and again subjected to reaction with ammonium orthophosphate as seen above.

The phosphate copolymer may be washed, once or more if required, with small portions of the same ammonium hydro-orthophosphate solution, then dried to the attainment of a constant weight. The phosphate copolymer thus obtained (called Flogucide) appears as a colorless vitreous mass, easily soluble, comprising linear chains and branched and/or crosslinked chains as illustrated hereto. In the final product no cyanuric acid derivatives nor hexamethylenediamine derivatives are detected. This product has not been previously described in literature.

As an alternative to the phosphate salt, the chloride may be suitably replaced by other salts like sorbate, stearate or dehydroacetate. In this case, the chloride of the copolymer is reacted with an alkaline solution, like sodium or potassium hydroxide, and with the corresponding acid under conditions that are usual and well known to a person skilled in the art.

The copolymers obtained through the process of the present invention exhibit a widely variable distribution of

the molecular weight and likely consist in linear chains and branched and/or crosslinked chains of various length having the following formulas:

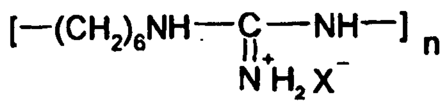

and

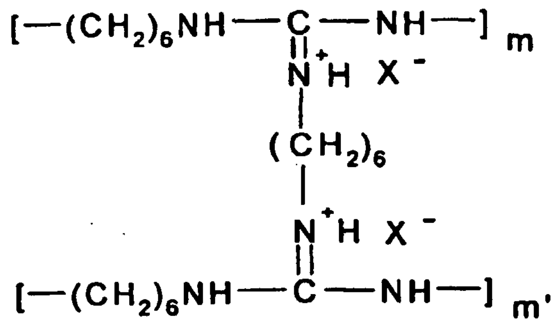

wherein $X^-$ is present in the salt form or absent in the free base form, and when present represents, alone or in a pair, chloride, dibasic phosphate, monobasic phosphate, sorbate, stearate or dehydroacetate, whereas the indexes n, m and m' are indefinite, equal or different thereamong, but such as to give the copolymer a molecular weight sufficiently high to ensure the flocculating and antimicrobial activity of the copolymer.

The ratio between linear chains and cross chains is equally variable and it depends, as above, from the relative ratios of diamine (e.g. hexamethylenediamine) and of guanidine chloride salt. An increase of the excess of hexamethylenediamine with respect to the stoichiometric value of the guanidine chloride, or the presence of an additional crosslinking agent, results in an increase of the crosslinking and of the intrinsic viscosity of the final product with or without loss of solubility. Excesses of ethylenediamine of from 5% to 15% produce copolymers soluble in water or in aqueous solutions exhibiting a maximum intrinsic viscosity of 0.21 dl/g. On the contrary, ethylene diamine excesses of from 30% to 50% produce insoluble copolymers in form of gels swelling in water and exhibiting characteristics of cationic resins having bactericidal activity.

Intermediate excesses result in mixtures of soluble and insoluble copolymers exhibiting intrinsic viscosity greater than 0.21 dl/g or 0.36 dl/g.

Moreover, the copolymers of the invention are characterized by irrelevant contents of residual hexamethylenediamine, present in an amount not higher than the 0.1%, but preferably of the 0.05% or the 0.01% and by the absence of the hydrocyanic acid derivatives (ammeline and ammelides).

From the applicative viewpoint, the copolymers according to the invention exhibit wide-range sterilizing properties, in particular bactericidal, antiviral, antimycotic and generally antifouling activities. For antifouling activity it is meant activity of prevention and elimination of any biological contamination form, due not only to the abovecited microorganisms but e.g. to algae, molds, parasites, crustaceans, mussels, plants, etc. Moreover, they exhibit flocculating activity towards the organic, protein and solid material contained in the water, as well as the capability of binding in solution and eliminating ions of metal, such as chrome, cadmium, copper, magnesium, nickel, lead, zinc, iron, aluminum, phosphor, with yields ranging from 20 to 90% depending on the metal. These properties make them suitable for water potabilisation, industrial or civil waste water purification, air sterilization in living and industrial environments and in clinic-hospital environments and structures, sterilization of solid materials and surfaces thereof, prevention and elimination of biological contamination. Applied on any solid material or surface or included in decorative or protective coating products, the solid state copolymers of the invention are capable, into contact with air, to absorb microorganisms and the dusts contaminated thereby. When applied on solid surfaces into contact with fresh or salt water, they exert a double antifouling and corrosion-preventing function. By virtue of their toxic activity on the cell membrane, they cause the destruction of any absorbed microorganism. In the form of phosphate salt, the product according to the invention is generally defined "Flogucide".

This product is not volatile and, when used diluted in water treatment, it exerts a sterilizing, flocculating and metal ion absorbing/binding action, without however altering the organoleptic characteristics of the water, like color or odor. Its maximum non-toxic concentration is of about 8 mg/l, whereas the effective concentration for water sterilization is of about 1.5 mg/l. The significant difference between maximum tolerated concentration and effective sterilizing concentration makes the product particularly suitable for water potabilisation.

The copolymers of the invention in form of insoluble gels find application as filtering ion-exchange resins exhibiting sterilizing activity.

For the treatment of drinking or waste waters, the copolymers of the invention are formulated in liquid or solid compositions; the former in form of solution, emulsion, suspension, the latter in form of granulate, powder, tablet, capsule, microcapsule or gel. The amounts used are such as to result in concentrations of from 0.5 to 2.0 mg/l, preferably of from 1.0 to 1.5 mg/l. Preferably these compositions will contain suitable diluents, adjuvants, stabilizers or supports.

Compositions containing the copolymers of the invention, in form of solution, emulsion, suspension, gel or paste, are also used in the sterilizing and antifouling treatment of solid materials or surfaces thereof. These compositions are capable of impregnating said materials or of forming decorative or protective coatings on said surfaces, exerting also a preventive and protective action against corrosion due to biological, atmospheric agents or to contact with fluids like river water or marine water.

These compositions may be in form of paints, enamels, latexes, resins or any other form of coating, based on or containing polymers like polychlorobutadiene, polytrichlorobutadiene, polyvinylacetate, poly(meta)acrylates or other polymer, copolymer or mixtures thereof. The cationic copolymer of the invention is contained in these latter products in an amount ranging from 0.5% to 65%, or from 0.5% to 8% or from 3% to 7% by weight, and it gives them, upon product solidification, one or more of the bactericidal, antiviral, antimycotic, antifouling activities. Moreover, the resistance to corrosion typical of the compositions of the invention effectively preserves such activities, which therefore are not lost over time.

Materials apt to be treated with the above compositions are glass, sintered glass, fiber glass, activated carbon and inorganic materials, cellulose, paper, fabrics, plastic or other solid polymer, metal, wood, hides or building material. These materials, once impregnated or surface-treated, are used for the preparation of marketable items of interest in the different technological fields where the attainment of sterile conditions or the prevention of any form of biological contamination due to bacteria, viruses, fungi, yeasts, molds, parasites, crustaceans, mussels, plants or other organisms are a priority. In a preferred embodiment of the invention the material is a filtering material, like e.g. sintered glass, paper, cellulose, activated carbon or resin, suitable for the sterilizing filtration of liquids or gases and used for the preparation of filtering elements. Conversely, the material is a plastic or metallic material, surface-treated and used for the production of containers or sections of gas- or liquid-transporting ducts. These filtering elements or ducts find application in the plants supplying and transporting drinking water or other food-use liquid, as well as in aeration systems of living, industrial, environments or in health and hospital structures.

In the methods of purification, sterilisation and potabilisation of water according to the invention, the liquid is treated with the copolymers or the compositions above, and/or filtered through a filtering element and/or conveyed through ducts as described above.

In particular, the technique of purification and sterilisation of spring water by means of the phosphate salts of the copolymers of the invention comprises the following steps:

- the preliminary bactericidal-flocculating treatment, carried out with the copolymer solution in a concentration of 0.5-1.5 mg/l, in order to obtain the required hygiene safety levels of the plants, to prevent the internal growth of the microorganisms and to increase the effectiveness of the water filtration;
- the coagulating of the flocculated water obtained with a mineral coagulating agent;
- the clarifying of the yielded water obtained by settling;
- the filtering of the water;
- the final bactericidal treatment of the water obtained with the copolymer solution of the maximum non-toxic value of 1.5 mg/l, yet anyhow not lower than 1.0 mg/l.

The technique of purification and sterilisation of waste waters, already preliminarily purified biologically (after the second settling) with the use of the phosphate copolymer of the invention, provides the bactericidal treatment with the same copolymer carried out in three consecutive steps with a total dosage of 1.0-1.5 mg/l. The waste waters are treated in three stages with amounts of 0.5-0.7 mg/l, 0.25-0.4 mg/l and 0.25-0.4 mg/l, each alternated with a 20-30 min interval, and subsequently filtered on a standard filter. In order to minimize environmental contamination due to the introducing into the environment of waters purified with the copolymers of the invention, the waters are further treated with an anionic flocculating agent or filtered on activated carbon to neutralize or remove the residual concentration of the copolymer. The index of coli-bacteria in the waters thus purified meets the official standards for the waste waters destined to return to the environment. Apart from its sterilizing action, the copolymer purifies water from the heavy metal ions, removing up to the 90% thereof depending on the kind of metal.

In the methods of purification and sterilisation of air or other gases in accordance with the invention, the air is filtered with filtering elements pretreated with polyhexamethyleneguanidine salts and/or conveyed into piping pretreated with polyhexamethyleneguanidine salts. Preferably, the filtering elements and the piping are the abovedescribed ones.

The technique of purification and sterilisation of air in living, industrial or hospital environments comprises the following optional steps:

- a filtering through the filters containing a copolymer of the invention. During this step, up to the 99.95-99.97% of the pathogenic bacteria, *Legionella* included, remain on the filters and are destroyed into contact with the copolymer;
- the bactericidal treatment of cooling liquids, specifically of water, used in air conditioning and heat exchange systems with a copolymer solution at concentrations of 1.0-1.5 mg/l. This treatment excludes the chance of spreading infections, due e.g. to *Legionella,* in the cases of its presence in the air conditioning systems. The most resilient *Legionella* strain is eliminated by a copolymer solution of 1.1 mg/l. To date, the danger of contagion due to *Legionella,* via the inhaling of infected particles, is very high in any air conditioning system.

The invention is further detailed by means of the following exemplary embodiments and applications thereof, given merely by way of illustration and without limitative purposes.

*Example 1.*

*Synthesis of polyhexamethyleneguanidine chloride according to the invention.*

1200 g guanidine crystals are placed in a 10-1 volume carbon steel reactor with 1088 g ammonium chloride crystals. The crystal mixture is mixed to homogeneity and brought at a 200°C temperature until complete melting of the crystals. The ammonia released in gaseous form during the reaction is conveyed, through the cooled coil of the condenser placed above the reactor, in an aqueous solution of 33.1% orthophosphoric acid. The reaction is carried on under stirring until ammonia production and release is over. During the second stage of the synthesis process, the molten state hexamethylenediamine is introduced in the mixture in two times, through a heated pipe. In a first stage there are used 2360 g hexamethylenediamine (obtaining with this amount the equimolar ratio between the hexamethylenediamine and the guanidine) which are brought, in a suitable vessel, at a 60-80°C temperature through a heating bath. The same temperature is held until completed melting of the hexamethylenediamine (melting temperature=40-43°C). Then, hexamethylenediamine is uniformly introduced in the reactor through a pipe heated for 2 hours, under a pressure of 0.3 $kg/cm^2$ and the mixture is mixed for 5 hours. A further amount (118 g) of hexamethylenediamine, corresponding to an excess on the equimolecular value of 0.05/1 (5%), is heated in the vessel placed in a heating bath and introduced in the mixture at the same rate. The reactor temperature is held at 200°C. During the reaction, ammonia release and hexamethylenediamine evaporation occur. The vapors thus produced are condensed and separated from the ammonia through passage in a water-cooled condenser placed above the reactor. The ammonia outletted from the cooling coil is conveyed to and absorbed in an aqueous solution of 33.1% orthophosphoric acid. At the same time, the condensed hexamethylenediamine returns, by percolation, into the reaction mixture, thereby keeping the hexamethylenediamine/guanidine molar ratio constant. Upon completing the introduction of the hexamethylenediamine, the temperature is raised up to 250°C, keeping the mixture under stirring for 10 hours until ammonia release is over. The total amount of ammonia released during the second stage of the polycondensation is of 726 g, of the theoretically computed amount.

Subsequently, the molten state substance is transferred into a carbon steel container, letting it cool to room temperature. Lastly, the solidified product having an amber-like appearance is mechanically granulated, fragmented and weighed. The amount of chloride of the polyhexamethyleneguanidine copolymer thus synthesized is of 3694 g; the content of the residual hexamethylenediamine is lower than the 0.1%; no cyanuric acid derivatives are detected. The intrinsic viscosity of the final product is of 0.07 dl/g. The product obtained is granulated by electric mill and used to prepare 500 ml of 50% aqueous solution.

*Synthesis of phosphate salt*

250 ml aqueous solution of 40% ammonium hydro-orthophosphate, produced via the neutralizing of (33.1%) orthophosphoric acid with ammonia, are heated up to 60° C and mixed to 500 ml of the aqueous solution of 50% polymethylguanidine chloride prepared as described above, keeping the mixture under magnetic stirring. After a 1-hour stirring, the mixture is placed in a settler and rested until separation of two distinct phases. The viscous bottom layer is separated and watered down to attain a 500 ml volume, then the mixture is added again with 250 ml 40% ammonium hydrophosphate solution, always under magnetic stirring. After a further 1-hour stirring, there is separated the bottom layer, which is again washed twice with 50 ml of the 40% ammonium hydrophosphate solution. For the washing, the following technique is applied: the settler is shaken, the mixture is rested and the bottom layer of copolymer is separated. The copolymer layer washed, is vacuum dried at 60° C. The product obtained is Flogucide.

To evaluate the sterilizing and flocculating power of the polyhexamethyleneguanidine phosphate of the invention, a 0.1% aqueous solution thereof is used. To 1 1 water containing $7x10^5$ *E.coli* bacteria, 1 ml solution as prepared

above is added under stirring, thereby reaching a polyhexamethyleneguanidine phosphate concentration of 1 mg/l. After 50-60 min of contact, no more living bacterial cells are detected in the solution; this fact confirms the high sterilizing power of the sample placed in the water. In a second test, 1 1 water containing $8.8x10^6$ particles of average size of 0.08 mm is mixed to 1 ml polyhexamethyleneguanidine phosphate solution as prepared above, thereby reaching a 1 mg/l concentration. Then, a sample of the suspension is analyzed at the microscope (included in Opton's 'Microvideomat' set) to detect the amount of the particles and establish their average size. The observed values will be of $7.3x10^6$ and 0.34 mm, respectively. The increase in the average size of the particles concomitantly to the decrease of the particle number confirms the flocculating power of the sample.

According to the computations, from 3694 g polyhexamethyleneguanidine chloride 6.8 1 of 50% aqueous solution thereof may be prepared. To carry out the substitution reaction of the $Cl^-$ ions with the $HPO_4^{2-}$ ones on such a volume of solution of 50% copolymer, 8.2 1 solution of 40% ammonia hydro-orthophosphate are required. Since the total volume of ammonium hydro-orthophosphate obtained through the neutralization of the aqueous solution of 33.1% orthophosphoric acid with the ammonia produced during the synthesis process is of 15.8 1, such a quantity is more than sufficient to carry out the substitution reaction of the $Cl^-$ ions with the $HPO_4^{2-}$ ions.

*Example 2.*

The method is similar to that described in the preceding example 1, except for the amount of the excess of hexamethylenediamine, in this case of 236 g, which leads to obtain an excess with respect to the hexamethylenediamine/guanidine equimolar ratio of 0.1/1 (10%). The total amount of ammonia released during the second stage of the reaction is of 759 g and it is of the computed theoretical amount. The amount by weight of the polyhexamethyleneguanidine chloride obtained is of 3778 g, its intrinsic viscosity is of 0.10 dl/l. The hexamethylenediamine content in the polyhexamethyleneguanidine chloride is lower than the 0.1%, whereas in the polyhexamethyleneguanidine phosphate no traces of hexamethylenediamine are detected. The sterilization of 1 l of water containing $5x10^5$ *E.coli* bacteria occurs within the 40-50 minutes subsequent to the dilution of 1 mg of sample obtained. The amount of particles present in 1 l of water with 1 mg of the diluted sample decreases from $8.8x10^6$ to $6.8x10^6$ with the concomitant increase of the average size thereof from 0.08 mm to 0.60 mm.

According to the computations, from 3778 g of polycondensation product 6.9 1 aqueous solution of 50% polyhexamethyleneguanidine chloride can be prepared. To carry out the substitution reaction of the $Cl^-$ ions with the $HPO_4^{2-}$ ones in this volume of 50% copolymer solution, 8.3 1 of 40% ammonium hydro-orthophosphate solution are required. The total volume of ammonium hydro-orthophosphate obtained by neutralization of the aqueous solution of orthophosphoric acid with ammonia is of 16.3 1 and it is more than sufficient to carry out the substitution reaction of the $Cl^-$ ions with the $HPO_4^{2-}$ ions.

*Example 3.*

The method is similar to that described in the preceding example 1, except from the excess amount of hexamethylenediamine, in this case of 354 g, which leads to obtain an excess with respect to the hexamethylenediamine/guanidine equimolar ratio of 0.15/1 (15%). The total amount of ammonia released during the second stage of the reaction, 793 g, corresponds to that expected by the theoretical computation. The amount by weight of the polihexamethylenediamine chloride is of 3862 g and its intrinsic viscosity of 0.21 dl/l. The content of hexamethylenediamine in the polihexamethylenediamine chloride is lower than 0.1%.

Instead, in the sample of polyhexamethylenediamine phosphate of the invention, no traces of hexamethylenediamine are detected.

The sterilization of 1 l of water containing $8x10^5$ *E. coli* bacteria cells is carried out by diluting 1 mg of the copolymer obtained according to the abovedescribed method and treating the water for 30-40 min. Following said treatment there were also observed: a decrease of the in-liquid particles from $8.8x10^6$ to $6.2x10^6$ and a concomitant increase of their sizes from 0.08 mm to 0.87 mm.

From 3862 g of polycondensation product there may be prepared 7.1 l of 50% aqueous solution thereof. For the substitution of the $Cl^-$ ions with the $HPO_4^{2-}$ ions in said volume, 8.5 l solution of 40% ammonium hydro-orthophosphate are required. The total volume of ammonium hydro-orthophosphate, obtained with the neutralization of the aqueous solution of 33.1% orthophosphoric acid with the ammonia produced during the synthesis, is of 16.8 1, and therefore it is sufficient to carry out the substitution reaction of the $Cl^-$ ions with the $HPO_4^{2-}$ ions.

Example 4.

The method used is similar to that described in the preceding example 1, except for the use of guanidine carbonate.

Prior to the synthesis, 600 g guanidine and 915 g guanidine carbonate are mixed, or the guanidine is completely substituted by the guanidine carbonate. In the case of a complete substitution 1830 g guanidine carbonate are used.
Then, the ammonia in gaseous form and the carbon dioxide released during the reaction are conveyed to the outside of the reactor, through the cooled coil placed above the reactor. The amount by weight of the polyhexamethyleneguanidine chloride is of 3862 g and its intrinsic viscosity is of 0.07 dl/l. In the polyhexamethyleneguanidine phosphate of the invention no traces of hexamethylenediamine are detected.

Example 5.

The method is similar to that described in example 1, except for the use of cyanoguanidine.
Prior to the synthesis 600 g guanidine and 427 g cyanoguanidine are mixed, or the guanidine is completely substituted by the cyanoguanidine. In the case of a complete substitution 854 g cyanoguanidine are used.
The weight of the polyhexamethyleneguanidine chloride of the invention is of 3694 g, and its intrinsic viscosity of 0.07 dl/l. In the polyhexamethyleneguanidine phosphate of the invention no traces of hexamethylenediamine are detected.

Example 6.

With the method indicated in example 3, a solution of 0.1% polyhexamethyleneguanidine phosphate was prepared. 1 l of river water having a load of suspended substances of 15 mg/l, a chrominance of 30° and a coli-bacteria index of $1.6x10^4$, was then kept under stirring by a mechanical stirrer.
The water, stirred at a 300 rpm rate, was mixed, over 2 min, to 1.5 ml of the previously prepared 0.1% solution, reaching a final concentration of 1.5 mg/l. After having brought the mixing rate to 100 rpm for 5 min, 7 mg of aluminum sulfate were introduced in the mixture. Then, the whole was mixed for 20 min at a 40 rpm rate.
The mixture was decanted for 30 min, then separated from the precipitate and filtered with a suitable sand filter having 0.5-1.2mm grains. The polyhexamethylamine phosphate concentration was measured and found to be of 0.5 mg/l. To the filtered water 1 ml of the previously prepared 0.1% solution was added, reaching a final concentration of 1.5 mg/l, and all of it was decanted for 1 hour.
Then, a chemico-bacteriological analysis on the purified water was carried out. The detected residual aluminum concentration is of 0.03 mg/l, the concentration of suspended substances is of 1 mg/l and the chrominance is of 8°. No traces of *E. coli* cells were detected.
The results of the experiments carried out demonstrated the occurred flocculation of the river water following the introduction therein of the polyhexamethyleneguanidine phosphate of the invention in an amount of 1.0-1.5 mg/l. Moreover, there was demonstrated an entailed coagulation of the water with the aluminum sulfate added in the concentration of 5-8 mg/l, which ensures a decrease of the residual aluminum concentration up to the 50-80% in the water previously decanted and filtered. The total sterilization of the water with a concentration of the copolymer of the invention of 1.5 mg/l is carried out in a time ranging from 30 to 60 minutes, depending on the initial amount of microorganisms present in the water.

*Example 7.*

With the method indicated in example 3, a solution of 0.1% polyhexamethyleneguanidine phosphate was prepared. Then, 1 l of waste water having an *E.coli* index of $5x10^7$ was rapidly mixed to 0.5 ml of the previously prepared 0.1% solution, reaching a final concentration of copolymer of the invention of 0.5 mg/l. The whole was decanted for 20 min. Then, further 0.25 ml of the 0.1% polyhexamethyleneguanidine phosphate solution were added, thereby reaching a final concentration of the copolymer of the invention of 0.75 mg/l. All of it was rapidly mixed and then decanted for 20 min. Then, further 0.25 ml of the 0.1% solution were added, thereby reaching a final concentration of the copolymer of the invention of 1 mg/l. After 20 min the water thus prepared was filtered through a granulated expanded clay filter, having a granule size of 1-2 mm, at a 10 m/h rate. The heavy metal ion concentration was analyzed with the inductively bound plasma atomic-emissive spectrum. The resulting waste water purification level was of the 80% for cadmium ions, the 60% for copper ions, the 20% for magnesium ions, the 15% for nickel ions, the 90% for lead ions, the 70% for zinc ions, the 60% for iron ions, the 50% for aluminum ions and the 20% for phosphor ions.
The *E. coli* index of the filtered water does not exceed the $10^3$ limit. Then, the water was filtered through an activated carbon filter at a 5 m/h rate. No trace of the polyhexamethyleneguanidine phosphate of the invention was detected in the water thus filtered.
In an experiment analogous to the preceding one, 1 mg of the previously prepared 0.1% solution was introduced, in a single step, in 1 1 waste water obtaining a final concentration of 1.0 mg/l. The *E. coli* index detected in the water thus prepared exceeds the $10^3$ limit, it being in fact of $2x10^4$. The results of this experiment indicate that the copolymer

of the invention should not be introduced in a single step.

*Example* 8.

The method is alike that reported in the preceding example 2. By mixing, 575 g polyhexamethyleneguanidine chloride, reduced to pieces of a 2-3 cm size, are diluted in 1150 g hot water. Continuing to mix energetically, 25.8 ml epichlorohydrin and 50 ml KOH are added, dropwise, and the mixture is rested for 8 hours until separation of two phases, the first one containing the aqueous solution of KCl and the second one the dense aqueous solution of the copolymer. Upon percolation of the salt solution the copolymer concentration is measured. The intrinsic viscosity of the solution is of 0.35 dl/g and it continues to increase for 3-5 days, reaching the value of 1.35 dl/g. The product concentration is of the 10%. Then, there is prepared the aqueous solution of the 0.1% copolymer which is used as flocculating agent to purify chrome-containing waste waters. 1 1 of tanning industry waste water (containing 57 mg/l chromium oxide, 2700 mg/l suspended particles, 431 mg/l albumin, BOD index of 2450 $mgO_2$/l, COD index of 710 $mgO_2$/l) is mixed to 1-2 ml aqueous solution of 0.1% copolymer with a rate of 120 rpm for 1 min. Continuing to mix, 200 mg of aluminum sulfate coagulating agent are introduced. After 2 min the rate is lowered to 40 rpm and it is mixed for other 20 minutes. Then, the mixture is rested for 30 min and the abovementioned indexes are measured again. The new measuring detects the following values: 1.3-1.5 mg/l chromium oxide, 250-350 mg/l suspended particles and 170-190 mg/l albumin, and at 180-190 $mgO_2$/l of 85-97 $mgO_2$/l. The water purified without use of the flocculating agent exhibits the following values: the chromium oxide is in the amount of 2,5 mg/l, the suspended particles are in the amount of 1250 mg/l, the albumin is in the amount of 290 mg/l, the BOD index is of 395 $mgO_2$/l and the COD index is of 210 $mgO_2$/l.

*Example 9.*

The method is alike that described in example 1. 1 l aqueous suspension is prepared, containing 20 g phosphate sample or chloride sample of polyhexamethyleneguanidine, 18.6 g acrylic latex, 8.3 g fluorocarbon resin, 1.5 g antifoam. 1.7 g fiberglass paper, of specific weight of 70 g/$m^2$, of dimensions of 10 $cm^2$ are immersed in the suspension. Then, the suspension-impregnated sample of paper is placed under vacuum to remove the excess thereof, and dried in a stove at a 140°C temperature. Then, the paper thus treated is used as a filter, to filter, at a 201/min rate, air contaminated by *E. coli* at a concentration of $1.5 \cdot 10^7$ cells/ml. The filter efficiency (bacteria trapping) is of the 99.97%. Moreover, in order to evaluate its bactericidal power the filter is placed for 24 hours on a Petri plate at a 37°C temperature, and the development of bacterial colonies is subsequently checked. After 24 hours there can be checked that no case of *E.coli* growth has been produced. The results obtained witness the total inactivation (100%) of the bacteria adsorbed by the filter containing the the polyhexamethyleneguanidine phosphate or chloride of the invention. Samples of air contaminated by $3.5 \cdot 10^5$ *Legionella* cells/ml are sterilized using the same method. To evaluate the bactericidal power of the filter with regard to *Legionella* cells, the filter, used to purify air, is placed into a vessel, in a yeast- and carbon-containing agar culture broth based on a system buffered with selective additions of substance. The incubation in this environment is protracted for 14 days at a 35° C temperature, with a daily checking of the results.

The filter efficiency (bacteria trapping) is of the 99.98% and no living bacterial cells are found in the water used for the washing of the filter.

*Example 10.*

15 g activated carbon are mixed in 300 ml aqueous solution of the copolymer obtained with the method reported at the preceding example 1, at a 10 g/l concentration, into the magnetic mixer for 1 hour at a 22°C temperature. Then, the activated carbon is separated on filter from the polymer solution, washed several times with distilled water in a 750 ml amount for each washing, for 1 hour, stirring it well until no copolymer trace is detected in the washing water. The carbon is again separated on filter and it is vacuum dried in a stove, at a 3 mmHg pressure and a 40°C temperature. To evaluate its bactericidal power, the dried coal is placed into an air filter, which is then used to filter, at a 1 m/hour rate, the air contaminated by *E.coli* at a concentration of $9.5 \cdot 10^4$ cells/ml. The efficiency of the filter (bacteria trapping) is of the 99.95%. In the water, used for the washing of the carbon extracted from the filter, no living microorganism cells are detected.

*Example 11.*

The method is alike that described in example 2. 575 g polyhexamethyleneguanidine chloride fragmented into pieces of a 2-3 cm size are diluted in 1150 g hot water, under stirring. To the 50% solution thus obtained, there are added, dropwise and stirring vigorously, 180 ml epichlorohydrin and 200 ml aqueous solution of KOH (128.8 g), then resting the solution for 8 hours. At the end, there is obtained a mass in form of an easily granulated solid gel. The

granules, suitably swelled in water, are used to fill up a filtration column (Ø 25 mm, height 26 mm) to remove the soluble fraction thereof. The column thus prepared is used to filter a water sample containing $5 \cdot 10^4$ *E.coli* cells/ml, at a 3.7 cm/min rate. In 1 l of water thus filtered no microorganisms are detected.

3 $cm^3$ gel granules are placed in a Ø 25 mm filtration column in order to evaluate the dynamic exchange capacity thereof. This column is used to filter initially 200 ml of a 0.1 N NaOH solution at a 40°C temperature and at a 3 ml/min rate, then 300 ml distilled water. Thereafter, 200 ml of 0.1 N HCl solution are filtered at a 3 ml/min rate collecting the percolate in a vessel. Then, the Titrant reactant containing a 0.1 N NaOH solution is added slowly. The starting concentration of the acid solution is of 110 mg-equivalents/l, its post-filtration concentration is of 82 mg-equivalents/l. The dynamic exchange capacity is of 1.87 mg-equivalents/$cm^3$ and characterizes the product obtained as a perfect anion.

20 ml of clinoptilolite in form of 1.0-1.6 mm granules are placed in a 50% polyhexamethyleneguanidine chloride solution into a ceramic vessel. Then, the solution is decanted, accurately retaining the granules with a spatula. Then the granules are treated, stirring them lightly with a spatula, with the epichlorohydrin mixture added dropwise and sodium hydroxide. Subsequently, the granules are vacuum dried, at a 40°C temperature, washed again to neutral pH of the percolated washing waters, vacuum dried again and placed into the filtration column. The clinoptilolite thus modified is placed into a filter used for the filtering, at an 1 m/hour rate, of air contaminated by *E.coli* at a concentration of $1.2 \cdot 10^4$ cells/ml. The filter efficiency (bacteria trapping) is of the 99.95%. The clinoptilolite sample is extracted from the filter and washed. In the water used for the washing no microorganisms are found.

<u>*Example 12.*</u>

A 0.1% hexamethylene guanidine phosphate or chloride solution was prepared according to the method illustrated in example 3.

Subsequently, sterile distilled water was contaminated with bacteria of the *Legionella* strain, at a $10^6$ cells/ml concentration. Then, to the contaminated water the aboveindicated solution was added, to a final concentration of 1.5 mg/l. The whole was mixed for 1 hour and then nebulized in a 1000 ml sterile glass vessel. A Ø 30 mm sterile carbon steel plate-indicator was suspended in the vessel and kept under stirring with a magnetic stirrer for 1 hour. Then, the plate was removed and washed with sterile distilled water. The procedure was repeated with another sterile steel plate, the stirring was effected for 8 hours and then the plate was removed and washed with sterile distilled water.

Any presence of living microorganisms in the water used for the washing of the plates was tested by selectively inoculating water samples in a suitable buffered culture medium, containing agar, yeast and carbon, and incubating all at 35 °C for a maximum period of time of 14 days. Any presence of living *Legionella* bacterial cells in the culture was tested daily. No presence of these cells was detected.

<u>*Example 13.*</u>

A 5% hexamethylene guanidine phosphate or chloride solution according to the method illustrated in example 3 was prepared

A Ø 30 mm degreased carbon steel plate was treated with the aboveindicated solution, then vacuum dried in a stove at 40°C. The bactericidal power of the copolymer of the invention was tested by placing the treated plate in a hermetically sealed 500 ml vessel, containing air infected with *Legionella* strain bacteria. The air was kept under stirring with a magnetic stirrer. The experiment was repeated and the treated plates were removed at different time intervals. Then, each plate was washed in sterile distilled water and any presence of living bacterial cells of the *Legionella* strain was tested according to the method illustrated in example 12.

No significant amounts of living bacterial cells were detected in the culture media; in fact, after a 24-hour incubation period of the plate, a decrease in the concentration of *Legionella* strain living bacterial cells of from $2x10^4$-$8x10^4$ cells/ml to 40-90 cells/ml was observed.

<u>*Example* 14.</u>

A 40% hexamethylene guanidine phosphate or chloride aqueous solution obtained according to the method illustrated in example 3 was mixed to enamel to a 5% final concentration. The enamel obtained was passed thrice on the surface of a Ø 30 mm degreased carbon steel plate, then the plate was vacuum dried in a stove at 40°C.

The bactericidal power of the copolymer of the invention was tested with the method illustrated in example 13.

No significant amounts of living bacterial cells were detected in the culture media; in fact, after a 24-hour incubation period of the treated plate, a decrease in the concentration of *Legionella* strain living bacterial cells of from $1x10^4$-$4x10^4$ cells/ml to 15-80 cells/ml was observed.

*Example 15.*

A polymer composition was prepared by mechanically mixing for 30. min 100 molar (mass) parts of polyvinylacetate aqueous dispersion, with 0.195 molar parts of 50% poly-sodium aluminophosphate solution. To this composition, there were added, continuing to mix, 8 molar parts of tetraethoxysilane and 3 molar parts of hexamethylene guanidine phosphate or chloride of the invention.

The obtained enamel was passed thrice on the surface of a Ø 30 mm degreased carbon steel plate, the plate was then vacuum dried in a stove at 40°C.

The bactericidal power of the copolymer of the invention was tested with the method illustrated in example 13.

No significant amounts of living bacterial cells were detected in the culture media; in fact, after a 24-hour incubation period of the treated plate, a decrease in the concentration of *Legionella* strain living bacterial cells of from $1.5x10^4$-$7.5x10^4$ cells/ml to 150-180 cells/ml was observed.

*Example 16.*

36.5 g polyhexamethyleneguanidine chloride, obtained according to the method indicated in example 3, were diluted in 70 ml of water at 55°C. This solution was added, dropwise, to 500 ml 20% sodium hydroxide solution.

There were prepared two solutions, containing respectively:

- 5.5 g polyhexamethyleneguanidine base according to the invention in 275 ml distilled water;
- 4.5 g polychlorobutandiene isomer in 225 ml chloroform.

The solutions were mixed at room temperature and the mixture was brought to pH 10 with sodium hydroxide. The obtained mixture was mixed for 10 min until obtaining a dark-colored mixture.

The chloroform-containing bottom phase was removed, washed twice or thrice with distilled water and dried on $CaCl_2$. The solution was filtered and softened in a rotary evaporator until decreasing 2-fold or 3-fold the volume thereof.

The obtained solution was nebulized on both the surfaces of a Ø 30 mm degreased carbon steel plate, and the plate was then vacuum dried in a stove at 40°C. The bactericidal power of the copolymer was evaluated according to the method illustrated in example 13.

No significant amounts of living bacterial cells were detected in the culture media; in fact, after a 24-hour incubation period of the treated plate, a decrease in the concentration of *Legionella* strain living bacterial cells of from $5x10^4$-$9x10^4$ cells/ml to 30-60 cells/ml was observed.

The strength and the effectiveness of the coating used on the plate were tested on two plates treated as indicated above, placing one plate for two months in a vessel containing river water and the other one plate for two months in a vessel containing marine water. Periodic checks performed on the plates detected no chipping or reduction of the coating.

Coating permeability was evaluated by weighing the plate prior to and after the treatment with the copolymer of the invention, and then by weighing a treated plate and an untreated plate after a 24-hour in-water immersion thereof. The weight of the treated plate is unvaried, demonstrating that the coating does not absorb water, nor it dissolves therein.

The copolymer of the invention protects the plate material from corrosion and from biofouling on the treated surface. The corrosion rate of the plate treated with the copolymer of the invention in fresh water or marine water is of 0, whereas the corrosion rate of the untreated plate is of 0.00985 $g/m^2$ in fresh water and of 0.0128 $g/m^2$ in marine water as indicated by the data of the following table (the corrosion rate was computed on the basis of the weight variation of treated and untreated plates, following immersion thereof in fresh or marine water):

| | FRESH WATER | | MARINE WATER | |
|---|---|---|---|---|
| | weight prior to contact, g | weight after contact*, g | weight prior to contact, g | weight after contact*, g |
| Copolymer-treated plate | 22.4291 | 22.4291 | 22.4291 | 22.4291 |
| Untreated plate | 22.3661 | 22.1216 | 22.3658 | 22.0471 |
| * the corrosion products were eliminated prior to plate weighing. | | | | |

The growth levels of the biological organisms on the treated plate were evaluated by placing the plates treated

with the copolymer of the invention in two vessels containing river water and marine water, respectively, both waters exhibiting a concentration of chlorophyll-containing algae of $2.3x10^6$ cells/ml. No algal growth was detected on both plates, and it was observed that, after 3-5 days of incubation, the algae present lose their pigment and precipitate. No algal adhesion was detected on the plates. In the control experiment, carried out likewise but with untreated plates, there was detected algal adhesion onto the surface of the examined plates and on the corrosion products. Moreover, survival of the algae was detected over the entire incubation time (of 14 days).

In order to evaluate the bactericidal power of the treated plate (having a coating of the weight of about 0.2675 g), said plate was placed in a vessel containing water contaminated with *E. coli* bacteria at a concentration of $12.9x10^7$ cells/ml and mixed, subsequently, for 30 min. The concentration of living *E. coli* observed after 30 min is of $10^3$ cells/ml, whereas that observed after 40 min is of 10 cells/ml.

Coatings comprising the compositions of copolymer polyethylene guanidine base of the invention and of polychlorobutadiene are used as such to cover air and water ducts, containers for marine water or river water and to protect water-contacting surfaces, like e.g. those of watercrafts or of buildings.

<u>*Example* 17.</u>

A sample of polyhexamethyleneguanidine base, obtained with the method indicated in example 16, is neutralized with an organic, inorganic and polymeric acid, so as to obtain the respective polyhexamethyleneguanidine salts.

*Synthesis of polyhexamethyleneguanidine sorbate of the invention:*

18 g polyhexamethyleneguanidine chloride, obtained with the method indicated in example 2, were diluted in 35 ml water. 5 ml water containing 5 g sodium hydroxide were added. Then, the viscous mass of polyhexamethyleneguanidine in form of base was separated, washed twice with 20 ml water and mixed with 11 g crystallized sorbic acid. Then, the obtained mass was dehydrated in a stove under vacuum and, subsequently, pulverized so as to obtain polyhexamethyleneguanidine sorbate powder. The resulting macromolecule retains the properties both of the sorbic acid and of the polyhexamethyleneguanidine base, and it is a powerful antiseptic. This substance can be deposited on various surfaces, giving an elevated antimicrobial power thereto.

*Preparation of a 20% polyhexamethyleneguanidine phosphate solution of the invention:*

117 g polyhexamethyleneguanidine chloride, obtained by the method indicated in experiment 2, were diluted in 350 ml hot water. Then, 60 ml water containing 60 g potassium hydroxide were added. The settled solution was separated and the obtained viscous mass of polyhexamethyleneguanidine base was washed thrice with 300 ml water. Subsequently, under stirring, 40 g (24 ml) concentrated orthophosphoric acid were added dropwise, until obtaining a clear polyhexamethyleneguanidine phosphate solution. The solution was brought to a 20% polyhexamethyleneguanidine phosphate concentration by adding water.

By substituting the sodium hydroxide (80 g 50% aqueous solution) to the potassium hydroxide, there is obtained polyhexamethyleneguanidine phosphate in form of paste that can be dried in air or in a stove under vacuum, pulverized and used for the preparation of a 20% solution. The use of potassium broadens the choice of the products and methods useful for the synthesis of the copolymer, allowing, moreover, to obtain polyhexamethyleneguanidine phosphate in liquid form.

*Synthesis of polyhexamethyleneguanidine chloride in pure form:*

700 g polyhexamethyleneguanidine chloride obtained by the method indicated in experiment 2 were diluted in 1400 ml hot water. Under stirring, 180 ml water containing 180 g NaOH were added. Then, the settled solution was separated and the white viscous mass of polyhexamethyleneguanidine base was washed thrice with 300 ml of water at 50°C. Upon cooling to room temperature, the residual water was percolated. Then, dropwise and under stirring, about 350 ml concentrated HCl were added until obtaining a clear viscous solution at pH 5, containing about 75% polyhexamethyleneguanidine chloride. The maximum concentration of hexamethylenediamine detectable in the product of experiment 2 is 1%, whereas in the 75% solution obtained in the present experiment the hexamethylenediamine concentration does not exceed the 0.1%.

*Synthesis of polyhexamethyleneguanidine stearate:*

To 100 ml 6% potassium salt aqueous solution, kept under vigorous stirring, there were added 28.5 g crystallized stearic acid in powder, and, dropwise, 53 ml aqueous solution of 33% polyhexamethyleneguanidine chloride obtained

by the method indicated in example 1. In the obtained suspension, the polyhexamethyleneguanidine stearate crystallizes, in form of spherical granules. Then, the product obtained was added to rubber mixtures to catalyze the cure reaction, and therefore the obtained rubber can be used in various protective coatings.

*Synthesis of polyhexamethyleneguanidine dehydroacetate:*

18 g polyhexamethyleneguanidine chloride obtained by the method indicated in example 2 were diluted in 35 ml water. Then, 5 ml water containing 5 g sodium hydroxide were added. Then, the viscous mass of polyhexamethyleneguanidine base was separated, washed twice in 20 ml of water, and mixed to 17 g crystallized dehydroacetic acid. Then, all of it was dried in a stove under vacuum and the polyhexamethyleneguanidine dehydroacetate was obtained. Dehydroacetic acid possesses fungicidal and bactericidal properties. The macromolecule thus obtained comprises the properties of two markedly antiseptic substances acting via different mechanisms, thereby becoming an antiseptic substance exhibiting a synergic sterilizing effect. This substance can be deposited on any surface, giving the latter an elevated antimicrobial power.

*Example 18.*

70.5 g polyhexamethyleneguanidine chloride obtained by the method illustrated in example 1 were mixed to 146 ml 38% formaldehyde solution and to 125 ml 0.01N borax solution. The mixture was heated in a heating bath for 6-8 hours until complete evaporation of the formaldehyde so as to obtain the methoxyl derivative of polyhexamethyleneguanidine chloride. The product obtained may be employed in hide tanning processes. After the complete evaporation of the formaldehyde (followed by the disappearance of its typical odor from the solution), the temperature was lowered to 60°C and 72 molar parts of β-naphtol or of α-naphthyl amine. All was incubated for other two hours at 60°C. The resin thus obtained was washed, to eliminate residues of β-naphthol or of α-naphthyl amine unused in the reaction, and treated with diazonium salt.

In the case of use of β-naphthol the diazonium salt was prepared by diluting 25 molar parts of sulphonyl acid in 65 ml 2N sodium hydroxide and then adding 10 molar parts of sodium nitrite in 120 ml water. Then the mixture was cooled to 10°C and there were introduced, under stirring, 130 ml of 2N HCl. Then the diazonium salt precipitate was mixed, for 30 min, with the basic solution of the β-naphthol derivative of the polyhexamethyleneguanidine chloride (41.6 molar parts of derivative in 225 ml of sodium hydroxide 2N solution). Then, 125 molar parts of NaCl were added and the mixture was refrigerated for 1 hour. The precipitate obtained, which is the dyeing derivative of the polyhexamethyleneguanidine chloride, was filtered, dried and used as naphthol derivative for the yellow dyeing of hides. The pigmentation of the hides is fast to wet- and dry-wiping, to light and to organic solvents. In the case of use of α-naphthyl amine the diazonium salt was prepared by diluting 17 molar parts of sulphonyl acid in 42 ml 2N sodium hydroxide and then adding 7 molar parts of sodium nitrite in 85 ml water. Then the mixture was placed on ice. The diazonium salt precipitate was then mixed with 41.4 molar parts of naphthyl amine derivative of the polyhexamethyleneguanidine chloride and with 170 ml of 1N HCl. Then, sodium hydroxide 2N solution was added, until reaching the basic reaction. The mixture was decanted for 2-3 hours and then the precipitate was washed and dried so as to obtain the naphthyl amine derivative, dyeing, of polyhexamethyleneguanidine chloride.

The derivative thus obtained can be used for the terracotta-red dyeing of hides. The pigmentation of the hides is fast to wet- and dry-wiping, to light and to organic solvents.

## Claims

**1.** A process for the preparation of hexamethylenediamine- and guanidine-based branched and/or crosslinked copolymers, wherein

a) molten state hexamethylenediamine is uniformly added over a period of time of 1.5 to 3 hours to equimolecular amounts of molten state guanidine chloride and the mixture kept at the melting temperature of the mass under stirring from 3 to 5 hours;
b) additional amounts of molten hexamethylenediamine resulting in a molar excess of hexamethylenediamine with respect to the guanidine chloride of 0.05-0.50/1 (5%-50%) are added to the molten mass holding the same temperature in a maximum period of time of 60 min;
c) then the temperature is increased to about 250°C and held thereat until free ammonia production is over, and wherein
all the steps provide the condensing and the reintroducing of the hexamethylenediamine vapors in the reaction mixture; and optionally

d) the chloride of the copolymer thus obtained is cooled and granulated and/or
e) converted into another salt or into the corresponding base.

**2.** The process according to claim 1, wherein the guanidine chloride is produced by mixing guanidine or guanidine carbonate or cyanoguanidine or mixtures thereof to equimolecular amounts of ammonium chloride and heating the mixture thus obtained to a temperature of about 200°C, until formation of molten state guanidine chloride.

**3.** The process according to claim 1 or 2, wherein the chloride of the copolymer is converted into the corresponding base or into phosphate, sorbate, stearate, or dehydroacetate.

**4.** The process according to claim 3, wherein the chloride is converted into phosphate through reaction with an ammonium hydro-orthophosphate solution produced by absorption of the ammonia released during the polymerization process in an orthophosphoric acid solution until neutralization.

**5.** The process according to any one of the claims 1 to 4 wherein a soluble copolymer is produced by using a molar excess of hexamethylenediamine with respect to guanidine chloride of 0.05-0.15/1.

**6.** The process according to any one of the claims 1 to 4, wherein the insoluble copolymer having characteristics of a cationic gel swelling in water is obtained using a molar excess of hexamethylenediamine with respect to the guanidine chloride of 0.3-0.50/1.

**7.** The process according to any one of the claims 1 to 4, wherein the addition of a molar excess of hexamethylenediamine with respect to the guanidine chloride of from 0.15 to 0.50 is carried out in the presence of a diluent.

**8.** The process according to any one of the claims 1 to 7, comprising a further stage of crosslinking carried out with epichlorohydrin or equivalent crosslinking agent.

**9.** The process according to any one of the claims 1 to 8, wherein hexamethylenediamine is totally or partially substituted by another C2-C10 alkylenediamine.

**10.** A C2-C10 alkylenediamine- and guanidine-based branched and/or crosslinked copolymer obtainable through the process according to any one of the claims 1 to 9.

**11.** The copolymer according to claim 10, comprising linear chains of formula:

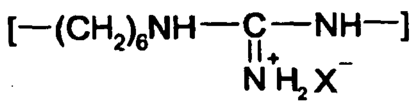

and branched and/or crosslinked chains of formula:

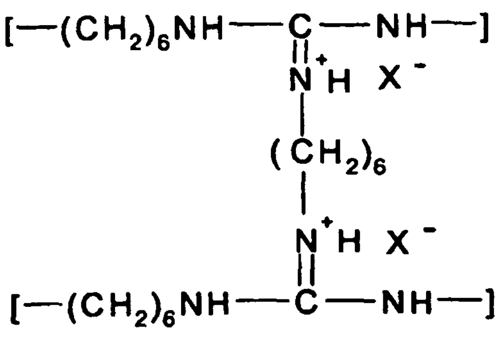

in form of salt or of base, wherein $X^-$, when present, represents, alone or in a pair, chloride, phosphate, sorbate, stearate, or dehydroacetate.

**12.** The copolymer according to claims 10 or 11, **characterized in that** it is soluble in an aqueous medium with a maximum viscosity of 0.21 dl/g or it is in form of gel insoluble swelling in an aqueous medium.

**13.** A flocculating and/or sterilizing and/or heavy metal ion binding composition for water purification and water potabilisation containing the cationic copolymer according to any one of the claims 10 to 12 and optionally one or more suitable diluents, adjuvants or supports.

**14.** The composition according to claim 13 in form of solution, emulsion, suspension or in form of granulate, powder, tablet, capsule, microcapsule or gel.

**15.** A sterilizing and/or antifouling composition for the treatment of materials or solid surfaces, comprising the copolymer according to any one of the claims 10 to 12 and one or more suitable diluents, adjuvants or supports.

**16.** The composition according to claim 15 in form of solution, emulsion, suspension, gel or paste, capable of impregnating said materials or of forming decorative or protective coatings on said surfaces.

**17.** The composition according to any one of the claims 15 to 16, **characterized in that** it is in form of paint, enamel, latex or resin.

**18.** The composition according to any one of the claims 13 to 17, having one or more of the bactericidal, antiviral, antimycotic, antifouling or anticorrosion activities.

**19.** A solid material, **characterized in that** it has been treated with the composition according to any one of the claims 15 to 17.

**20.** The material according to claim 19, **characterized in that** it is glass, sintered glass, fiber glass, carbon, inorganic material, cellulose, paper, fabric, plastic or other solid polymer, metal, wood, hide or building material.

**21.** The material according to claim 20, **characterized in that** it is a filtering material suitable for the sterilizing filtration of liquids or gases.

**22.** A cationic resin, **characterized in that** it consists of the insoluble copolymer in form of gel according to claim 12.

**23.** An article, made with or comprising the material according to any one of the claims 19 to 21, or the resin according to claim 22.

**24.** The article according to claim 23, **characterized in that** it is a filtering element suitable for treatment of gases or liquids or it is a piping for the transport of waters or of air.

**25.** A method of purifying and sterilizing air in living, or industrial environments, **characterized in that** the air is filtered through filtering elements and/or conveyed into piping according to claim 24.

**26.** A method of purification, sterilisation and potabilisation of waters, **characterized in that** the liquid is treated with the copolymer according to any one of the claims 10 to 12, or with the composition according to any one of the claims 13 to 14, and/or filtered through a filtering element and/or conveyed into ducts according to claim 24.

**27.** A method for waters potabilisation according to claim 26, comprising a preliminary bactericidal-flocculating treatment carried out with a copolymer solution of 0.5-1.5 mg/l, and an final bactericidal treatment of the water obtained with the copolymer solution of concentration equal of 1.0-1.5 mg/l.

**28.** A method for waste waters purification and sterilisation according to claim 27, comprising a bactericidal treatment with 1.0-1.5 mg/l of copolymer carried out in three consecutive steps with addition of the copolymer in amounts of 0.5-0.7 mg/l, 0.25-0.4 mg/l and 0.25-0.4 mg/l alternated with a 20-30 min period of time.

**29.** A method of sterilisation and antifouling and/or corrosion protecting treatment of solid materials or surfaces thereof **characterized in that** said materials are impregnated or surface-treated with the composition according to any one of the claims 15 to 18.

**30.** A method of sterilizing and antifouling treatment of hides or fabrics, **characterized in that** those are impregnated or surface-treated with the composition according to any one of the claims 15 to 18.

## Patentansprüche

**1.** Verfahren zur Herstellung von verzweigten und/oder vernetzten Copolymeren auf Hexamethylendiamin- und Guanidin-Basis, wobei

a) Hexamethylendiamin in geschmolzenem Zustand gleichmäßig über einen Zeitraum von 1,5 bis 3 Stunden zu äquimolaren Mengen an Guanidinchlorid in geschmolzenem Zustand zugegeben wird und das Gemisch 3 bis 5 Stunden lang unter Rühren bei der Schmelztemperatur der Masse gehalten wird;
b) zusätzliche Mengen an geschmolzenem Hexamethylendiamin, die zu einem molaren Überschuss an Hexamethylendiamin, bezogen auf das Guanidinchlorid, von 0,05 bis 0,50/1 (5% bis 50%) führen, über einen maximalen Zeitraum von 60 min zu der bei der gleichen Temperatur gehaltenen geschmolzenen Masse zugegeben werden;
c) dann die Temperatur auf etwa 250°C erhöht und dabei gehalten wird, bis die Bildung von freiem Ammoniak vorüber ist, und wobei
alle Schritte die Kondensation und Rückführung der Hexamethylendiamin-Dämpfe in das Reaktionsgemisch bereitstellen; und gegebenenfalls
d) das Chlorid des so erhaltenen Copolymers abgekühlt und granuliert wird und/oder
e) in ein anderes Salz oder in die entsprechende Base umgewandelt wird.

**2.** Verfahren nach Anspruch 1, wobei das Guanidinchlorid durch Einmischen von Guanidin oder Guanidincarbonat oder Cyanoguanidin oder Gemischen davon in äquimolare Mengen von Ammoniumchlorid und Erwärmen des so erhaltenen Gemischs auf eine Temperatur von etwa 200°C bis zur Bildung von Guanidinchlorid in geschmolzenem Zustand hergestellt wird.

**3.** Verfahren nach Anspruch 1 oder 2, wobei das Chlorid des Copolymers in die entsprechende Base oder in ein Phosphat, Sorbat, Stearat oder Dehydroacetat umgewandelt wird.

**4.** Verfahren nach Anspruch 3, wobei das Chlorid durch Umsetzen mit einer Ammoniumhydroorthophosphatlösung, welche durch Absorption des während des Polymerisationsverfahrens freigesetzten Ammoniaks in einer Orthophosphorsäurelösung bis zur Neutralisation gebildet wird, in Phosphat umgewandelt wird.

**5.** Verfahren nach einem der Ansprüche 1 bis 4, wobei ein lösliches Copolymer unter Verwendung eines molaren Überschusses an Hexamethylendiamin, bezogen auf Guanidinchlorid, von 0,05 bis 0,15/1 hergestellt wird.

**6.** Verfahren nach einem der Ansprüche 1 bis 4, wobei das unlösliche Copolymer, das die Eigenschaften eines kationischen Gels, das in Wasser quillt, aufweist, unter Verwendung eines molaren Überschusses an Hexamethylendiamin, bezogen auf das Guanidinchlorid, von 0,3 bis 0,50/1 erhalten wird.

**7.** Verfahren nach einem der Ansprüche 1 bis 4, wobei die Zugabe eines molaren Überschusses an Hexamethylendiamin von 0,15 bis 0,50, bezogen auf das Guanidinchlorid, in Gegenwart eines Verdünnungsmittels durchgeführt wird.

**8.** Verfahren nach einem der Ansprüche 1 bis 7, umfassend einen weiteren Vernetzungsschritt, der mit Epichlorhydrin oder einem äquivalenten Vernetzungsmittel durchgeführt wird.

**9.** Verfahren nach einem der Ansprüche 1 bis 8, wobei Hexamethylendiamin vollständig oder teilweise durch ein anderes $C_2$-$C_{10}$-Alkylendiamin substituiert ist.

**10.** Verzweigtes und/oder vernetztes Copolymer auf $C_2$-$C_{10}$-Alkylendiamin- und Guanidin-Basis, erhältlich durch das Verfahren nach einem der Ansprüche 1 bis 9.

**11.** Copolymer nach Anspruch 10, umfassend lineare Ketten der Formel:

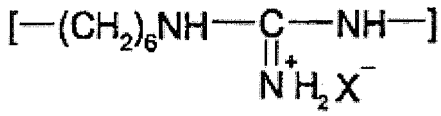

und verzweigte und/oder vernetzte Ketten der Formel:

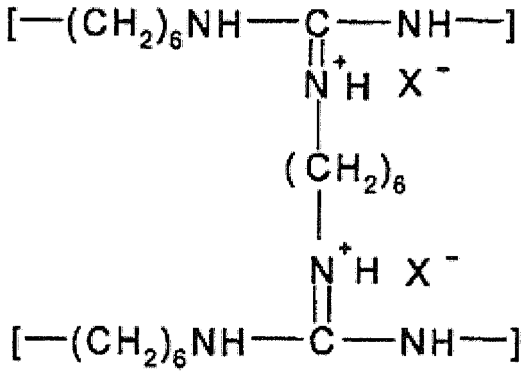

in Form eines Salzes oder einer Base, wobei $X^-$, falls vorhanden, allein oder in einem Paar Chlorid, Phosphat, Sorbat, Stearat oder Dehydroacetat bedeutet.

**12.** Copolymer nach den Ansprüchen 10 oder 11, **dadurch gekennzeichnet, dass** es in einem wässrigen Medium mit einer maximalen Viskosität von 0,21 dl/g löslich ist oder in Form eines Gels vorliegt, das unlöslich in einem wässrigen Medium quillt.

**13.** Flockungsmittel- und/oder sterilisierende und/oder Schwermetallionen bindende Zusammensetzung zur Wasserreinigung und Trinkbarmachung von Wasser, welche das kationische Copolymer nach einem der Ansprüche 10 bis 12 und gegebenenfalls ein oder mehrere geeignete Verdünnungsmittel, Hilfsstoffe oder Träger enthält.

**14.** Zusammensetzung nach Anspruch 13 in Form einer Lösung, Emulsion, Suspension oder in Form eines Granulats, Pulvers, einer Tablette, Kapsel, Mikrokapsel oder eines Gels.

**15.** Sterilisierende und/oder fäulnisverhindernde Zusammensetzung zur Behandlung von Materialien oder festen Oberflächen, umfassend das Copolymer nach einem der Ansprüche 10 bis 12 und ein oder mehrere geeignete Verdünnungsmittel, Hilfsstoffe oder Träger.

**16.** Zusammensetzung nach Anspruch 15 in Form einer Lösung, Emulsion, Suspension, eines Gels oder einer Paste, welche(s) zum Imprägnieren der Materialien oder zum Bilden dekorativer oder schützender Beschichtungen auf den Oberflächen in der Lage ist.

**17.** Zusammensetzung nach einem der Ansprüche 15 bis 16, **dadurch gekennzeichnet, dass** sie in Form eines Anstrichmittels, eines Emaillelacks, eines Latex oder Harzes vorliegt.

**18.** Zusammensetzung nach einem der Ansprüche 13 bis 17 mit einer oder mehreren bakteriziden, antiviralen, antimykotischen, fäulnisverhindernden oder korrosionsverhindernden Wirksamkeiten.

**19.** Festes Material, **dadurch gekennzeichnet, dass** es mit der Zusammensetzung nach einem der Ansprüche 15 bis 17 behandelt wurde.

**20.** Material nach Anspruch 19, **dadurch gekennzeichnet, dass** es Glas, gesintertes Glas, Fiberglas, Kohlenstoff, anorganisches Material, Cellulose, Papier, Gewebe, Kunststoff oder ein anderes festes Polymer, Metall, Holz, Haut

oder Baustoff ist.

**21.** Material nach Anspruch 20, **dadurch gekennzeichnet, dass** es ein Filtermaterial ist, das zur sterilisierenden Filtration von Flüssigkeiten oder Gasen geeignet ist.

**22.** Kationisches Harz, **dadurch gekennzeichnet, dass** es aus dem unlöslichen Copolymer in Form eines Gels nach Anspruch 12 besteht.

**23.** Gegenstand, hergestellt mit dem oder umfassend das Material nach einem der Ansprüche 19 bis 21 oder das Harz nach Anspruch 22.

**24.** Gegenstand nach Anspruch 23, **dadurch gekennzeichnet, dass** er ein Filterelement ist, das zur Behandlung von Gasen oder Flüssigkeiten geeignet ist, oder dass er eine Rohrleitung für den Transport von Wasser oder Luft ist.

**25.** Verfahren zur Reinigung und Sterilisation von Luft auf dem privaten oder gewerblichen Sektor, **dadurch gekennzeichnet, dass** die Luft durch Filterelemente filtriert und/oder in einer Rohrleitung nach Anspruch 24 befördert wird.

**26.** Verfahren zur Reinigung, Sterilisation und Trinkbarmachung von Wasser, **dadurch gekennzeichnet, dass** die Flüssigkeit mit dem Copolymer nach einem der Ansprüche 10 bis 12 oder mit der Zusammensetzung nach einem der Ansprüche 13 bis 14 behandelt und/oder durch ein Filterelement filtriert und/oder in Rohrleitungen nach Anspruch 24 befördert wird.

**27.** Verfahren zur Trinkbarmachung von Wasser nach Anspruch 26, umfassend eine vorläufige bakterizid-flockenbildende Behandlung, die mit einer 0,5 bis 1,5 mg/l Copolymerlösung durchgeführt wird, und eine abschließende bakterizide Behandlung des erhaltenen Wassers mit der Copolymerlösung in einer Konzentration von 1,0-1,5 mg/l.

**28.** Verfahren zur Reinigung und Sterilisation von Abwasser nach Anspruch 27, umfassend eine bakterizide Behandlung mit 1,0 bis 1,5 mg/l Copolymer, die in drei aufeinanderfolgenden Schritten unter Zugabe des Copolymers in Mengen von 0,5 bis 0,7 mg/l, 0,25 bis 0,4 mg/l und 0,25 bis 0,4 mg/l, mit jeweils einem Zeitraum von 20 bis 30 Minuten dazwischen, durchgeführt wird.

**29.** Verfahren zur sterilisierenden und fäulnisverhindernden Behandlung und/oder Korrosionsschutzbehandlung fester Materialien oder deren Oberflächen, **dadurch gekennzeichnet, dass** die Materialien mit der Zusammensetzung nach einem der Ansprüche 15 bis 18 imprägniert oder oberflächenbehandelt werden.

**30.** Verfahren zur sterilisierenden und fäulnisverhindernden Behandlung von Häuten oder Gewebe, **dadurch gekennzeichnet, dass** diese mit der Zusammensetzung nach einem der Ansprüche 15 bis 18 imprägniert oder oberflächenbehandelt werden.

## Revendications

**1.** Procédé pour la préparation de copolymères ramifiés et/ou réticulés à base d'hexaméthylènediamine et de guanidine, dans lequel

a) de l'hexaméthylènediamine à l'état de fusion est ajouté de manière uniforme pendant un laps de temps de 1,5 à 3 heures à des quantités équimoléculaires de chlorure de guanidine à l'état de fusion et le mélange est gardé à la température de fusion de la masse sous agitation pendant 3 à 5 heures ;
b) des quantités additionnelles d'hexaméthylènediamine en fusion résultant en un excès molaire d'hexaméthylènediamine par rapport au chlorure de guanidine de 0,05 à 0,50/1 (5 % à 50 %) sont ajoutées à la masse en fusion en maintenant la même température pendant un laps de temps maximum de 60 minutes ;
c) ensuite la température est augmentée à 250° C environ et maintenue à celle-ci jusqu'à ce que la production d'ammoniac libre soit terminée,
et dans lequel toutes les étapes ont pour effet la condensation et la réintroduction des vapeurs d'hexaméthylènediamine dans le mélange réactionnel ; et de manière optionnelle
d) le chlorure du copolymère ainsi obtenu est refroidi et granulé et/ou
e) converti en un autre sel ou en la base correspondante.

**2.** Procédé selon la revendication 1, dans lequel le chlorure de guanidine est produit en mélangeant de la guanidine ou du carbonate de guanidine ou de la cyanoguanidine ou des mélanges de celles-ci à des quantités équimoléculaires de chlorure d'ammonium et en chauffant le mélange ainsi obtenu à une température de 200° C environ, jusqu'à formation d'un chlorure de guanidine à l'état de fusion.

**3.** Procédé selon la revendication 1 ou 2, dans lequel le chlorure du copolymère est converti en la base correspondante ou en phosphate, sorbate, stéarate, ou déhydroacétate.

**4.** Procédé selon la revendication 3, dans lequel le chlorure est converti en phosphate par réaction avec une solution d'ammonium hydro-orthophosphate produite par absorption de l'ammoniaque libéré au cours du procédé de polymérisation dans une solution d'acide orthophosphorique jusqu'à neutralisation.

**5.** Procédé selon l'une quelconque des revendications 1 à 4 dans lequel un copolymère soluble est produit en utilisant un excès molaire d'hexaméthylènediamine par rapport au chlorure de guanidine de 0,05 à 0,15/1.

**6.** Procédé selon l'une quelconque de revendications 1 à 4, dans lequel le copolymère insoluble ayant les caractéristiques d'un gel cationique gonflant dans l'eau est obtenu en utilisant un excès molaire d'hexaméthylènediamine par rapport au chlorure de guanidine de 0,3 à 0,50/1.

**7.** Procédé selon l'une quelconque des revendications 1 à 4, dans lequel l'ajout d'un excès molaire d'hexaméthylènediamine par rapport au chlorure de guanidine de 0,15 à 0,50 est réalisé en présence d'un solvant.

**8.** Procédé selon l'une quelconque de revendications 1 à 7, comprenant une étape supplémentaire de réticulation réalisée avec de l'épichlorohydrine ou un agent de réticulation équivalent.

**9.** Procédé selon l'une quelconque des revendications 1 à 8, dans laquelle l'hexaméthylènediamine est entièrement ou partiellement substituée par une autre alkylènediamine C2-C 10.

**10.** Copolymère ramifié et/ou réticulé à base de guanidine ou d'alkylènediamine C2-C10 pouvant être obtenu par le procédé selon l'une quelconque des revendications 1 à 9.

**11.** Copolymère selon la revendication 10, comprenant des chaînes linéaires de formule :

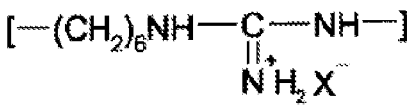

et des chaînes ramifiées et/ou réticulées de formule :

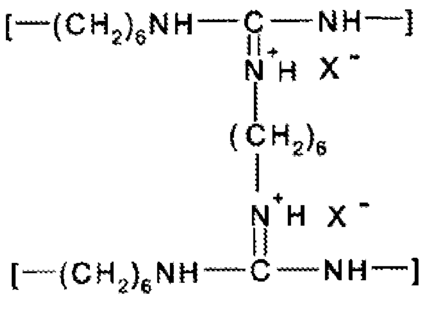

sous forme de sel ou de base, dans lequel $X^-$, lorsqu'il est présent, représente, seul ou en paire, le chlorure, le phosphate, le sorbate, le stéarate, ou le déhydroacétate.

**12.** Copolymère selon la revendication 10 ou 11, **caractérisé en ce qu'**il est soluble dans un milieu aqueux avec une viscosité maximale de 0,21 dl/g ou qu'il est sous la forme d'un gel insoluble gonflant dans un milieu aqueux.

**13.** Composition de floculation et/ou de stérilisation et/ou de séquestration d'ions métalliques lourds pour la purification de l'eau et la potabilisation de l'eau contenant le copolymère cationique selon l'une quelconque des revendications 10 à 12 et de manière optionnelle un ou plusieurs diluants, adjuvants ou supports adaptés.

**14.** Composition selon la revendication 13 sous la forme de solution, d'émulsion, de suspension ou sous la forme de granulé, de poudre, de comprimé, de capsule, de microcapsule ou de gel.

**15.** Composition de stérilisation et/ou d'antisalissure pour le traitement de matériaux ou de surfaces solides, comprenant le copolymère selon l'une quelconque des revendications 10 à 12 et un ou plusieurs diluants, adjuvants, ou supports adaptés.

**16.** Composition selon la revendication 15 sous la forme de solution, d'émulsion, de suspension, de gel ou de pâte, capable d'imprégner lesdits matériaux ou de former des revêtements décoratifs ou protecteurs sur lesdites surfaces.

**17.** Composition selon l'une quelconque des revendications 15 à 16, **caractérisée en ce qu'**elle est sous la forme de peinture, d'émail, de latex ou de résine.

**18.** Composition selon l'une quelconque des revendications 13 à 17, ayant une ou plusieurs activités parmi les activités bactéricides, antivirales, antimycosique, antisalissure ou anticorrosion.

**19.** Matériau solide, **caractérisé en ce qu'**il a été traité avec la composition selon l'une quelconque des revendications 15 à 17.

**20.** Matériau selon la revendication 19, **caractérisé en ce qu'**il est du verre, du verre fritté, de la fibre de verre, du carbone, un matériau inorganique, de la cellulose, du papier, du tissu, du plastique ou un autre polymère solide, du métal, du bois, du cuir ou du matériel de construction.

**21.** Matériau selon la revendication 20, **caractérisé en ce qu'**il est un matériau de filtration adapté pour la stérilisation de liquides ou de gaz.

**22.** Résine cationique, **caractérisée en ce qu'**elle consiste du copolymère insoluble sous la forme de gel selon la revendication 12.

**23.** Article, fait avec ou comprenant le matériau selon l'une quelconque de revendications 19 à 21, ou résine selon la revendication 22.

**24.** Article selon la revendication 23, **caractérisé en ce qu'**il est un élément de filtration adapté pour le traitement des gaz ou des liquides ou qu'il est un tuyau pour le transport des eaux et de l'air.

**25.** Méthode de purification et de stérilisation de l'air dans l'environnement ou dans un environnement industriel, **caractérisé en ce que** l'air est filtré par des éléments de filtration et/ou acheminé dans le tuyau selon la revendication 24.

**26.** Méthode de purification, de stérilisation et de potabilisation des eaux, **caractérisée en ce que** le liquide est traité avec le copolymère selon l'une quelconque des revendications 10 à 12, ou avec la composition selon l'une quelconque des revendications 13 à 14, et/ou filtré par un élément de filtration et/ou acheminé dans des canalisations selon la revendication 24.

**27.** Méthode pour la potabilisation des eaux selon la revendication 26, comprenant un traitement de floculation-bactéricide préliminaire réalisé avec une solution copolymère de 0,5 à 1,5 mg/l, et un traitement bactéricide final de l'eau obtenue avec la solution copolymère d'une concentration égale à 1,0 à 1,5 mg/l.

**28.** Méthode pour la purification et la stérilisation des eaux usées selon la revendication 27, comprenant un traitement bactéricide avec 1,0 à 1,5 mg/l de copolymère en trois étapes successives d'ajout de copolymère en des quantités de 0,5 à 0,7 mg/l, 0,25 à 0,4 mg/l et 0,25 à 0,4 mg/l alternées avec un laps de temps de 20 à 30 minutes.

**29.** Méthode de stérilisation et d'antisalissure et/ou traitement de protection de la corrosion des matériaux solides ou des surfaces de ceux-ci **caractérisée en ce que** lesdits matériaux sont imprégnés ou traités en surface avec la composition selon l'une quelconque des revendications 15 à 18.

**30.** Méthode de traitement de stérilisation et d'antisalissure des cuirs ou des tissus, **caractérisée en ce que** ceux-ci sont imprégnés ou traités en surface avec la composition selon l'une quelconque des revendications 15 à 18.